# EUROPÄISCHE PATENTSCHRIFT

(11) **EP 0 603 129 B1**
(45) Veröffentlichungstag und Bekanntmachung des Hinweises auf die Patenterteilung: **29.09.1999**
(21) Anmeldenummer: 93810854.5
(22) Anmeldetag: 07.12.1993
(51) Int. Cl.: C09B 19/00, D06P 3/76

(54) **Oxazinfarbstoffe, Verfahren zu deren Herstellung und deren Verwendung**
Oxazine dyes, process for their production and their use
Colorants oxaziniques, procédé de leur préparation et leur utilisation

(30) Priorität: 15.12.1992 CH 381992
(43) Veröffentlichungstag der Anmeldung: 22.06.1994
(73) Patentinhaber: Ciba Specialty Chemicals Holding Inc., 4057 Basel (CH)
(72) Erfinder: Birri, Hanspeter, CH-4133 Pratteln (CH); Hanika, Gerhard, Dr., D-79539 Lörrach (DE); Nicopoulos, Alex, CH-4052 Basel (CH)

(56) Entgegenhaltungen:
- EP-A- 0 038 736
- EP-A- 0 055 223
- CH-A- 642 987
- DE-A- 2 259 341
- DE-A- 2 631 207
- DE-A- 2 701 677
- DE-A- 2 929 285
- DE-B- 1 569 604
- GB-A- 1 190 235

## Beschreibung

Die vorliegende Erfindung betrifft neue Oxazinfarbstoffe, Verfahren zu ihrer Herstellung sowie ihre Verwendung zur Herstellung von lagerstabilen Flüssigformulierungen.

Basische Oxazinfarbstoffe für das Färben von z.B. Polyacrylnitril oder sauer modifizierten Polyestern sind in grosser Zahl z.B. aus der DE-AS 1 569 604 oder EP-A 0 038 736 bekannt. Eine wichtige Reinigungsoperation bei der Synthese dieser Farbstoffe besteht darin, die Verbindungen in Form eines schwerlöslichen Salzes aus dem Reaktionsmedium auszufällen, welches anschliessend direkt zu einer festen Handelsform weiterverarbeitet werden kann. An das ausgefällte Salz wird zudem die Anforderung gestellt, dass es leicht und vollständig in eine geeignete flüssige Formulierung überführbar ist. Zu diesem Zweck werden die kationischen Farbstoffe bisher oft umständlich und ökologisch nicht unbedenklich in Chlorozinkatsalze überführt, als solche ausgefällt und anschliessend durch Umsalzung z.B. in das entsprechende Acetat überführt.

Es wurden nun überraschend neue schwerlösliche Salze von basischen Oxazinfarbstoffen gefunden, die mit höherer Raum-Zeit-Ausbeute, d.h. einfacher und kostengünstiger, herzustellen und unproblematisch in Bezug auf ihr Abwasser sind und zudem leicht in eine flüssige Handelsform überführt werden können.

Gegenstand der vorliegenden Erfindung sind somit Verbindungen der Formel 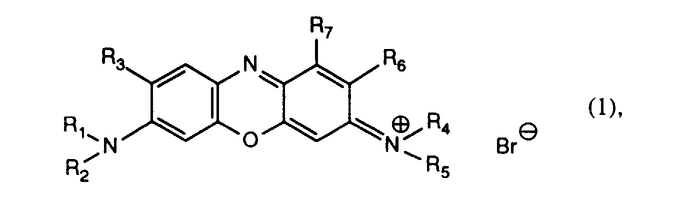 worin
R₁ Wasserstoff oder gegebenenfalls substituiertes Alkyl oder Phenyl,
R₂ Wasserstoff oder gegebenenfalls substituiertes Alkyl,
R₃ Wasserstoff, Alkyl oder Alkoxy,
R₄ Wasserstoff oder gegebenenfalls substituiertes Alkyl oder Phenyl,
R₅ Wasserstoff oder gegebenenfalls substituiertes Alkyl,
R₆ Wasserstoff, Alkyl oder Alkoxy und
R₇ Wasserstoff oder Alkyl bedeuten, oder R₆ zusammen mit R₇ einen ankondensierten Phenylring bilden.

Bedeuten R₁ oder etwaige andere Substituenten gegebenenfalls substituiertes Phenyl, handelt es sich um unsubstituiertes oder z.B. durch C₁-C₄-Alkyl, Cyclohexyl, C₁-C₄-Alkoxy, Cyano, Trifluoromethyl, Nitro oder Halogen substituiertes Phenyl. Beispiele für bevorzugte Phenylreste sind Phenyl und o-, m- oder p-Methylphenyl.

Bei R₁ oder etwaigen anderen Substituenten als gegebenenfalls substituiertem Alkyl handelt es sich z.B. um C₁-C₄-Alkyl, welches gegebenenfalls z.B. durch Halogen, Hydroxy, Cyano, Carbamoyl oder Alkoxy substituiert ist. Beispiele sind Methyl, Ethyl, n-oder iso-Propyl, n-, iso-, sec.- oder tert.-Butyl, Cyanoethyl und Hydroxyethyl. Vorzugsweise handelt es sich bei R₁ um einen unsubstituierten C₁-C₄-Alkylrest und besonders bevorzugt um Methyl oder Ethyl.

Bei R₃ oder etwaigen anderen Substituenten als Alkyl handelt es sich vorzugsweise um C₁-C₄-Alkyl und insbesondere um Methyl oder Ethyl.

Bedeuten R₃ oder etwaige andere Substituenten Alkoxy, handelt es sich vorzugsweise um C₁-C₄-Alkoxy, z.B. n- oder iso-Propoxy, n-, iso-, sec.- oder tert.-Butoxy oder, vorzugsweise, Methoxy oder Ethoxy.

Bevorzugt sind Verbindungen der zuvor angegebenen Formel (1), worin
R₁ Wasserstoff, Methyl, Ethyl, Hydroxyethyl, Phenyl oder o-, m- oder p-Methylphenyl,
R₂ Wasserstoff, Methyl oder Ethyl,
R₃ Wasserstoff, Methyl oder Methoxy,
R₄ Wasserstoff, Methyl oder Ethyl, Phenyl oder o-, m- oder p-Methylphenyl,
R₅ Wasserstoff, Methyl, Ethyl,
R₆ Wasserstoff, Methyl oder Methoxy und
R₇ Wasserstoff oder Methyl bedeuten.

Besonders bevorzugt sind Verbindungen der Formel 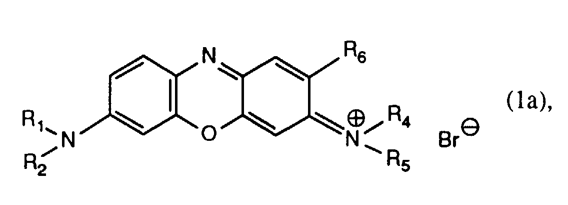 worin
R₁ und R₂ unabhängig voneinander je Methyl oder Ethyl,
R₄ Wasserstoff, Methyl, Ethyl, Phenyl oder o-, m- oder p-Methylphenyl,
R₅ Wasserstoff, Methyl, Ethyl, und
R₆ Wasserstoff, Methyl oder Methoxy bedeuten.

Eine besonders bevorzugte Ausführungsform der vorliegenden Erfindung betrifft die Verbindung der Formel

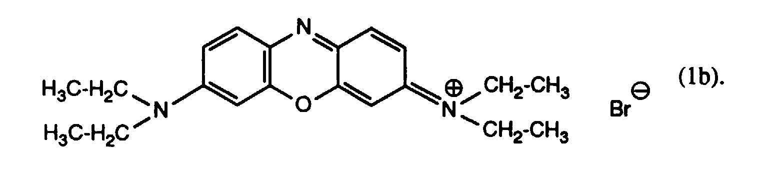

Die Verbindungen der Formel (1) können z.B. durch Umsalzen aus bekannten basischen Oxazinverbindungen, z.B. aus den entsprechenden Tetrachloroferraten, erhalten werden.

Es ist jedoch bevorzugt und stellt einen weiteren Gegenstand der Erfindung dar, dass man die Verbindungen der Formel (1) herstellt, indem man
a) eine Verbindung der Formel 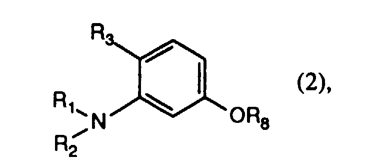 worin R₁, R₂ und R₃ jeweils die zuvor angegebene Bedeutung haben und R₈ für Wasserstoff oder gegebenenfalls substituiertes Alkyl steht, in einem Medium enthaltend ein dipolar-aprotisches Lösungsmittel, Bromwasserstoffsäure (HBr) und gegebenenfalls Wasser nitrosiert,
b) die erhaltene Nitrosoverbindung ohne Zwischenisolierung mit einer Verbindung der Formel 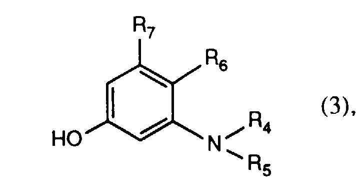 worin R₄, R₅, R₆ und R₇ die zuvor angegebene Bedeutung haben, kondensiert, und gegebenenfalls anschliessend
c) die Reaktionsmasse destillativ so einengt, dass das Farbstoffsalz ausfällt.

Das für die Nitrosierung gemäss Schritt a) des Verfahrens benutzte Medium enthält ein dipolares aprotisches Lösungsmittel, z.B. ein Ameisensäureamid wie insbesondere N,N-Dimethylformamid (DMF), und Bromwasserstoffsäure und kann ausserdem Wasser enthalten. Ein für die Nitrosierung bevorzugtes Medium besteht aus DMF, Bromwasserstoffsäure und Wasser und weist einen stark sauren pH-Wert, d.h. einen pH-Wert von z.B. ≤ 2, auf. Man verwendet als Nitrosierungsreagenz ein übliches anorganisches oder organisches Nitrit, z.B. ein Alkalimetallnitrit wie Natriumnitrit, welches fest oder vorzugsweise in Form einer wässrigen Lösung zudosiert wird.

Die Nitrosierung erfolgt vorzugsweise bei einer Temperatur unterhalb von 50°C und besonders bevorzugt bei Raumtemperatur, d.h. bei einer Temperatur von ca. 15 bis 30°C. Man verwendet in etwa stöchiometrische Mengen oder einen geringen Ueberschuss an Nitrit, bezogen auf die Verbindung der Formel (2).

Die Verbindungen der Formeln (2) und (3) sind an sich bekannt oder können in bekannter Weise erhalten werden.

Der Kondensationsschritt (b) des erfindungsgemässen Verfahrens wird analog zu bekannten Verfahren z.B. in einem das dipolar-aprotische Lösungsmittel, Bromwasserstoffsäure und gegebenenfalls Wasser enthaltenden Medium durchgeführt. Hierbei ist es bevorzugt, das 3-Aminophenol der Formel (3) in einem das dipolar-aprotische Lösungsmittel, vorzugsweise DMF, und gegebenenfalls Wasser enthaltenden Medium vorzulegen, den pH-Wert dieses Mediums mit einer anorganischen Säure, vorteilhaft mit Bromwasserstoffsäure, neutral bis leicht sauer, d.h. auf einen Wert von z.B. 4-7, einzustellen und dann die gemäss a) erhältliche Nitrosolösung langsam zulaufen zu lassen. Die für die Kondensationsreaktion (b) verwendete Temperatur beträgt z.B. 25 bis 120°C und vorzugsweise 50 bis 100°C.

Wird nach beendeter Kondensation eine Reaktionslösung erhalten, aus der sich der kationische Farbstoff nicht oder nur teilweise abscheidet, geht man bei der Aufarbeitung zweckmässig so vor, dass man zunächst aus dem sauren Kondensationsgemisch eine bestimmte Menge Lösungsmittel destillativ entfernt und dann die verbleibende Reaktionslösung abkühlt. Enthält z.B. das nach Beendigung der Kondensationsreaktion anfallende Reaktionsgemisch grössere Mengen DMF und Wasser, so unterwirft man dieses Reaktionsgemisch bevorzugt einer Vakuumdestillation und kühlt die eingeengte Reaktionslösung anschliessend z.B. auf Raumtemperatur ab. Das abdestillierte Lösungsmittel kann anschliessend zum Teil wieder bei der Nitrosierung eingesetzt werden.

Die Bromide der Formel (1) fallen im gewählten Reaktionsmedium praktisch quantitativ in kristalliner Form und in sehr guter Reinheit aus, während Verunreinigungen gelöst bleiben. Man erhält auf diese Weise einen sehr reinen Farbstoff von hoher Farbstärke ohne aufwendige Isolierungs- und Reinigungsoperationen.

Eine besonders bevorzugte Ausführungsform der vorliegenden Erfindung betrifft ein Verfahren zur Herstellung der Verbindung der Formel 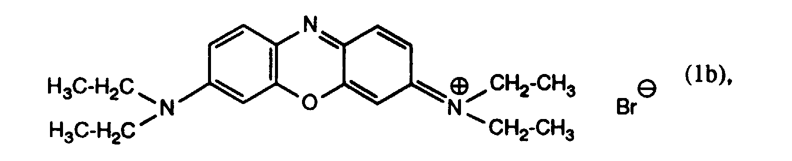 welches dadurch gekennzeichnet ist, dass man
a) eine Verbindung der Formel 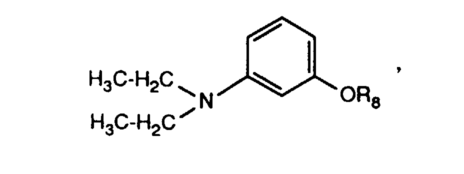 worin R₈ Wasserstoff, Methyl oder Ethyl bedeutet, in einem DMF, Bromwasserstoffsäure und Wasser enthaltenden Medium nitrosiert,
b) die erhaltene Nitrosoverbindung ohne Zwischenisolierung mit einer Verbindung der Formel 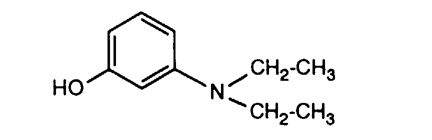 in einem DMF, Bromwasserstoffsäure und Wasser enthaltenden Lösungsmittelgemisch kondensiert, und
c) die Reaktionslösung destillativ so einengt, dass das Farbstoffsalz ausfällt.

Die erfindungsgemässen Verbindungen der Formel (1) bzw. (1a) oder (1b) eignen sich zum Färben oder Bedrucken von tannierten Cellulosefasern, Seide, Leder oder vollsynthetischen Fasern wie Acetatseide, Polyamidfasern oder sauer modifizierten Polyamid- oder Polyesterfasern, insbesondere jedoch von Polyacrylnitril wie auch Polyvinylidencyanid enthaltenden Fasern. Die erfindungsgemässen Verbindungen sind gut handhabbar und weisen z.B. eine gute Löslichkeit in Färbebädern auf. Nach üblichen Verfahren ausgefärbt ergeben sich jeweils Färbungen mit guten Allgemeinechtheiten.

Ein besonderer Vorteil der erfindungsgemässen Verbindungen der Formel (1) besteht darin, dass sie einfach in stabile Flüssigformulierungen überführt werden können.

Ein weiterer Gegenstand der Erfindung betrifft daher ein Verfahren zur Herstellung von stabilen flüssigen Formulierungen von Verbindungen der Formel 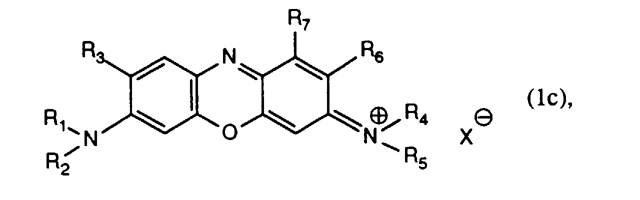 worin X^{⊖} für ein Monocarbonsäureanion steht, R₁ Wasserstoff oder gegebenenfalls substituiertes Alkyl oder Phenyl, R₂ Wasserstoff oder gegebenenfalls substituiertes Alkyl, R₃ Wasserstoff, Alkyl oder Alkoxy, R₄ Wasserstoff oder gegebenenfalls substituiertes Alkyl oder Phenyl, R₅ Wasserstoff oder gegebenenfalls substituiertes Alkyl, R₆ Wasserstoff, Alkyl oder Alkoxy und R₇ Wasserstoff oder Alkyl bedeuten oder R₆ zusammen mit R₇ einen ankondensierten Phenylring bilden, dadurch gekennzeichnet, dass man eine Verbindung der Formel 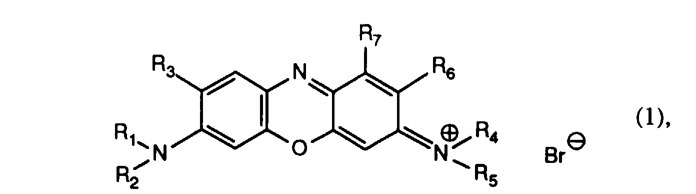 worin die Variablen R₁ - R₇ jeweils die zuvor angegebene Bedeutung haben, in einem Medium enthaltend die dem Anion X^{⊖} entsprechende Monocarbonsäure HX und ein Salz Ka^{⊕}X^{⊖} dieser Säure, worin Ka^{⊕} ein Kation bedeutet, behandelt, das ausfallende Bromid abfiltriert und gegebenenfalls überschüssige Carbonsäure HX z.B. destillativ entfernt und/oder die Farbstärke durch Zugabe von Wasser einstellt.

In der Formel (1c) gelten für die Variablen R₁, R₂, R₃, R₄, R₅, R₆ und R₇ jeweils die zuvor angegebenen Bedeutungen und Bevorzugungen. Das Anion X^{⊖} steht z.B. für das Anion einer unsubstituierten oder z.B. durch Hydroxy substituierten C₁-C₈-Alkansäure. Beispiele sind das Anion der Ameisensäure, Essigsäure, Propionsäure, Buttersäure, Pentansäure, Hexansäure, Heptansäure, Octansäure oder Milchsäure. X^{⊖} steht bevorzugt für das Anion der Ameisensäure oder Milchsäure und insbesondere bevorzugt für das Anion der Essigsäure, d.h. es handelt sich bei den Verbindungen der Formel (1c) bevorzugt um ein Formiat, Lactat oder insbesondere um ein Acetat.

Dementsprechend handelt es sich bei HX um eine der oben genannten Monocarbonsäuren und bei Ka^{⊕} X^{⊖} um das Salz einer der genannten Säuren.

Ka^{⊕} bedeutet z.B. ein Erdalkali- oder insbesondere ein Alkalimetall-Kation, z.B. das Lithium-Kation oder vorzugsweise das Natrium- oder Kalium-Kation.

Beispiele für bevorzugte Salze Ka^{⊕}X^{⊖} sind somit Natrium- und Kaliumformiat, Natrium- und Kaliumlactat und insbesondere Natrium- und Kaliumacetat.

Die Umsalzung des Bromids der Formel (1) in dem die Carbonsäure und deren Salz enthaltenden Medium wird z.B. bei einer Temperatur von 20 bis 150°C, vorzugsweise 30 bis 100°C und besonders bevorzugt 50 bis 80°C, durchgeführt. Das Reaktionsmedium sollte möglichst wasserfrei sein. Gegebenenfalls können wasserbindende Mittel, z.B. anorganische oder organische Säureanhydride wie etwa Acetanhydrid, wasserfreie Salze oder getrocknete M-Al-Silikate (Zeolite), zugesetzt werden, um den Wassergehalt des Reaktionsmediums zu verringern.

Eine bevorzugte Variante des Verfahrens besteht darin, dass man den bei der zuvor beschriebenen Herstellung des Bromids der Formel (1) anfallenden feuchten Presskuchen direkt in einem Medium enthaltend eine Carbonsäure HX, ein Salz Ka^{⊕}X^{⊖} dieser Säure und ein wasserbindendes Mittel bei erhöhter Temperatur, d.h. bei einer Temperatur von z.B. 50 bis 80°C, verrührt, das ausgefallene Bromid nach dem Abkühlen abfiltriert, überschüssige Carbonsäure HX abdestilliert und die Farbstärke der Lösung durch Zugabe von Wasser einstellt.

Eine besonders bevorzugte Ausführungsform der vorliegenden Erfindung betrifft ein Verfahren zur Herstellung einer stabilen flüssigen Formulierung der Verbindung der Formel 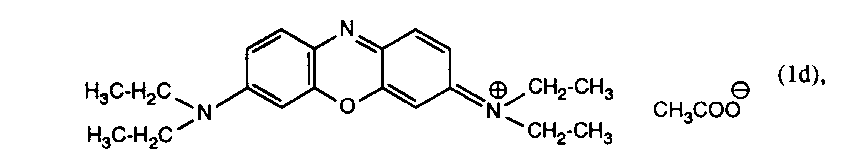 dadurch gekennzeichnet, dass man den bei der Synthese der Verbindung der Formel 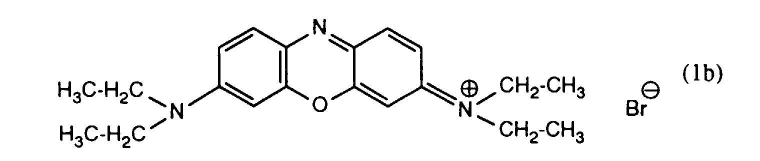 anfallenden feuchten Presskuchen des Produkts direkt in ein Essigsäure, Natrium- oder Kaliumacetat und Acetanhydrid enthaltendes Medium überführt und bei erhöhter Temperatur behandelt, anschliessend das ausgefallene Alkalibromid abfiltriert und gegebenenfalls überschüssige Essigsäure entfernt und/oder die Farbstärke der Lösung durch Zugabe von Wasser einstellt.

Die folgenden Beispiele veranschaulichen die Erfindung. Teile bedeuten Gewichtsteile, Prozente Gewichtsprozente. Die Temperaturen sind in °C angegeben.

### Beispiel 1:

A. Nitrosierung
   In ein geeignetes Reaktionsgefäss werden unter leichter N₂-Spülung 880 Teile DMF vorgelegt und 174 Teile 3-Diethylamino-1-ethoxybenzol bei Raumtemperatur hinzugefügt. Man addiert Wasser und Eis und lässt dann innerhalb von ca. 15 Minuten 158,8 Teile 100 %ige Bromwasserstoffsäure so zulaufen, dass die Temperatur ca. 20°C nicht übersteigt. Unter Konstanthalten der Temperatur (ca. 20°C) dosiert man innerhalb von ca. 1,5 Stunden 63,2 Teile Natriumnitrit in Form einer 40%igen wässrigen Lösung hinzu und lässt dann ca. 30 Minuten ausrühren.
B. Kondensation
   In ein geeignetes Reaktionsgefäss werden unter leichter N₂-Spülung 200 Teile DMF vorgelegt, 152,1 Teile 3-Diethylamino-phenol bei Raumtemperatur hinzugefügt und der pH-Wert mit Bromwasserstoffsäure auf einen leicht sauren Wert (pH ca. 5-6) eingestellt. Die erhaltene Lösung wird auf ca. 67-70°C erhitzt und die Nitroso-Lösung gemäss (A) innerhalb von ca. 3,5 bis 4 Stunden zulaufen gelassen. Man rührt ca. 45 Minuten bei 65-70°C nach und destilliert dann im Vakuum (Innentemperatur ca. 55-75°C, Innendruck ca. 100mbar) ein überwiegend aus DMF und Wasser bestehendes Destillat ab. Man lässt die verbleibende dunkelblaue Reaktionslösung auf Raumtemperatur abkühlen, wobei das Produkt praktisch quantitativ ausfällt. Nach dem Abfiltrieren, Waschen und Trocknen erhält man den Farbstoff der Formel 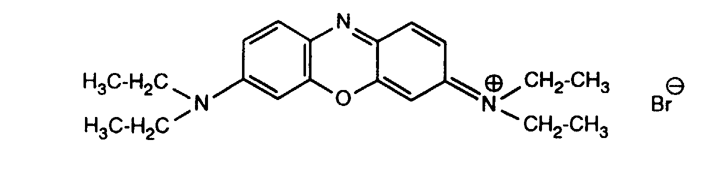 als salzarmes, reines Produkt mit hoher Farbstärke.
C. Herstellung einer flüssigen Handelsform
   In einem geeigneten Reaktionsgefäss werden 328 Teile des gemäss B. nach dem Abfiltrieren erhaltenen feuchten Nutschguts, 192 Teile Kaliumacetat, 720 Teile 100%ige Essigsäure und 160 Teile Acetanhydrid vorgelegt und verrührt. Das Gemisch wird auf 70-75°C aufgeheizt und ca. 4 Stunden bei dieser Temperatur gehalten. Anschliessend kühlt man das Reaktionsgemisch auf ca. 30°C ab und filtriert das entstandene Kaliumbromid ab. Das Filtrat wird anschliessend einer Vakuumdestillation unterworfen und ca. 540 g Essigsäure abdestilliert; anschliessend stellt man mit Wasser auf die gewünschte Farbstärke ein. Man erhält eine stabile flüssige Handelsform des Farbstoffs der Formel

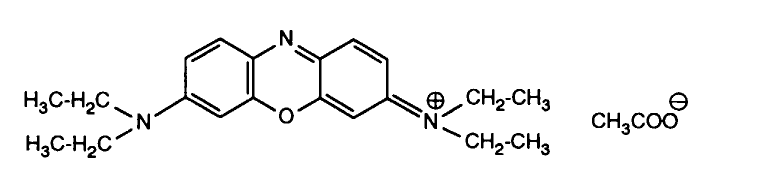

### Beispiele 2-11:

Analog zu dem in Beispiel 1 beschriebenen Verfahren lassen sich die folgenden Verbindungen herstellen und in eine flüssige Handelsform überführen:

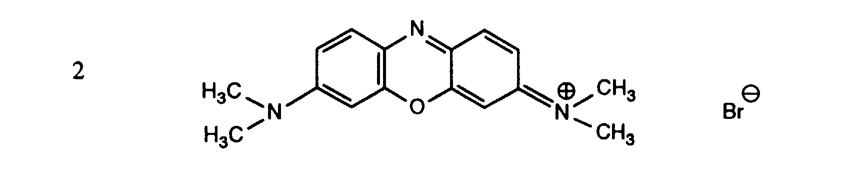

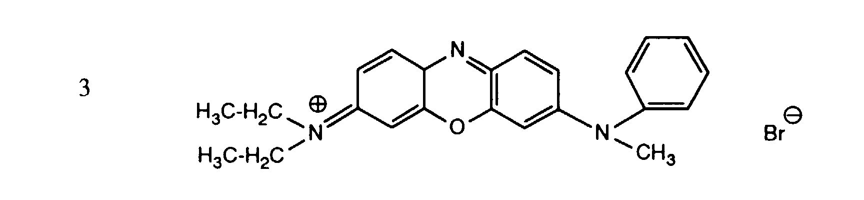

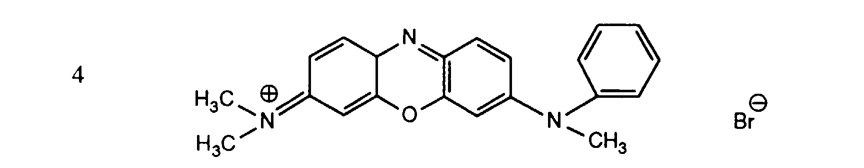

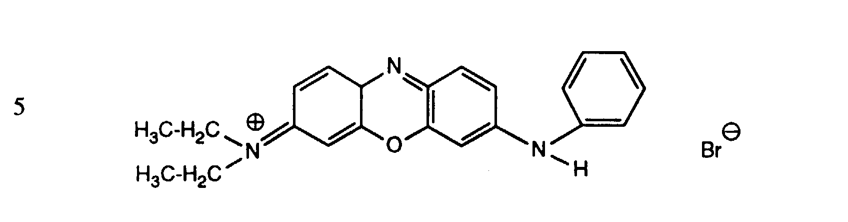

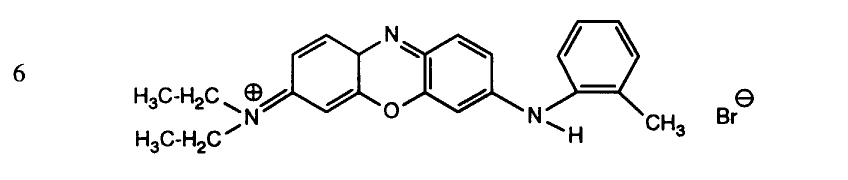

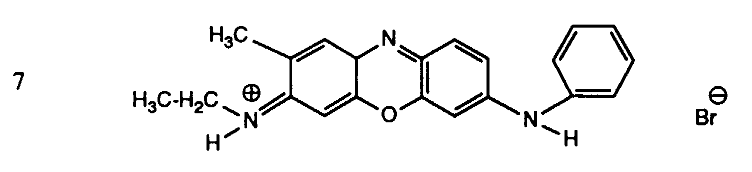

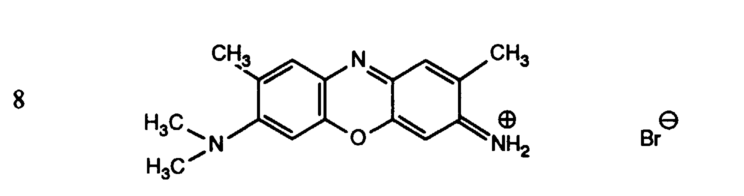

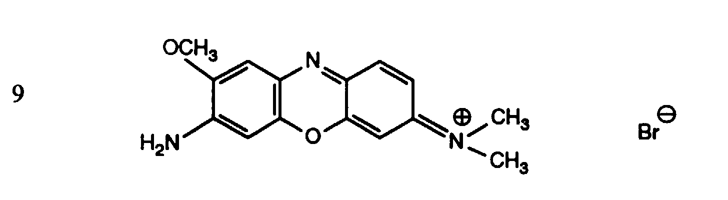

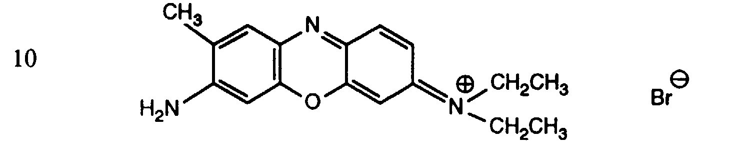

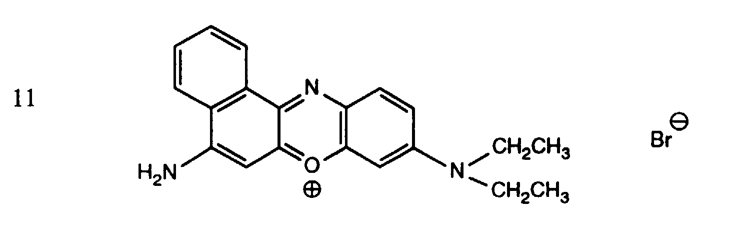

## Patentansprüche

1. Verbindungen der Formel

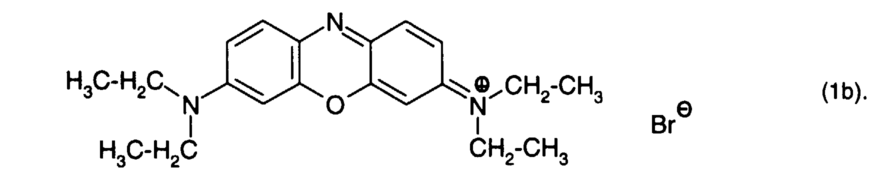

2. Verfahren zur Herstellung der Verbindung der Formel (1b) gemäss Anspruch 1, dadurch gekennzeichnet, dass man
a) eine Verbindung der Formel 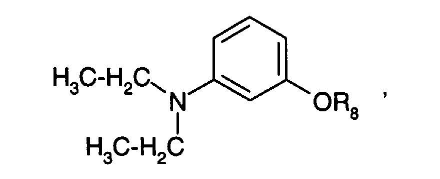 worin R₈ Wasserstoff, Methyl oder Ethyl bedeutet, in einem DMF, Bromwasserstoffsäure und Wasser enthaltenden Medium nitrosiert,
b) die erhaltene Nitrosoverbindung ohne Zwischenisolierung mit einer Verbindung der Formel 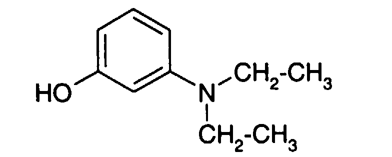 in einem einem DMF, Bromwasserstoffsäure und Wasser enthaltenden Lösungsmittelgemisch kondensiert, und
c) die Reaktionslösung destillativ so einengt, dass das Farbstoffsalz ausfällt.

3. Verwendung von Verbindungen der Formel (1b) gemäss Anspruch 1 zum Färben oder Bedrucken von tannierten Cellulosefasem, Seide, Leder oder vollsynthetischen Fasern.

4. Verwendung gemäss Anspruch 3 zum Färben oder Bedrucken von Polyacrylnitril oder Polyvinylidencyanid enthaltenden Fasern.

5. Verwendung von Verbindungen der Formel (1b) gemäss Anspruch 1 zur Herstellung von einer stabilen flüssigen Formulierung der Verbindung der Formel

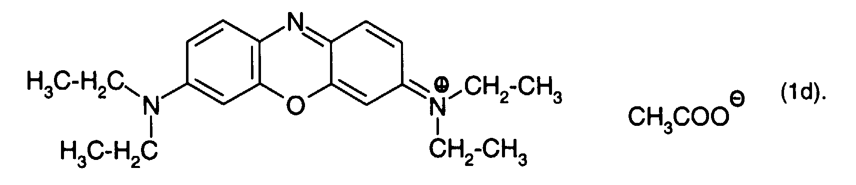

6. Verfahren zur Herstellung einer stabilen flüssigen Formulierung der Verbindung der Formel 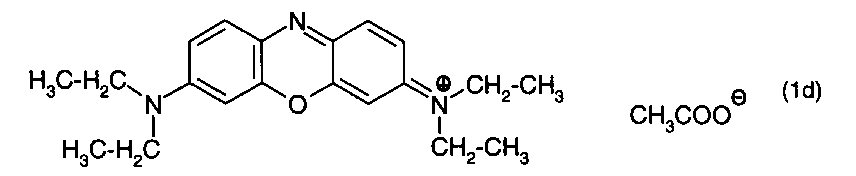 gemäss Anspruch 5, dadurch gekennzeichnet, dass man den bei der Synthese der Verbindung der Formel 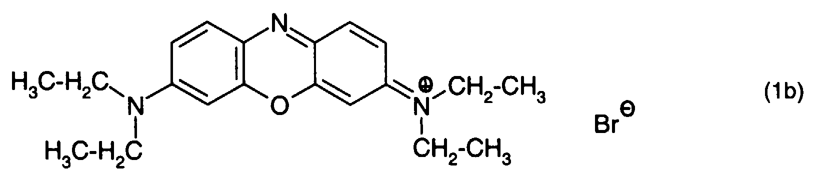 anfallenden feuchten Presskuchen des Produkts direkt in ein Essigsäure, Natrium- oder Kaliumacetat und Acetanhydrid enthaltendes Medium überführt und bei erhöhter Temperatur behandelt, anschliessend das ausgefallene Alkalibromid abfiltriert und gegebenenfalls überschüssige Essigsäure entfernt und/oder die Farbstärke der Lösung durch Zugabe von Wasser einstellt.

## Claims

1. The compound of the formula

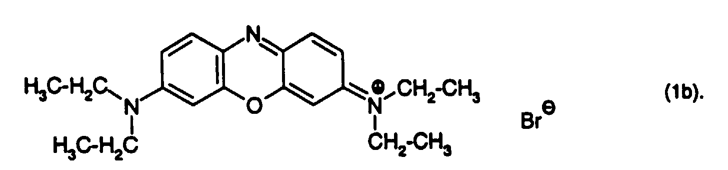

2. A process for preparing the compound of the formula (1b) according to claim 1, which comprises
a) nitrosating a compound of the formula 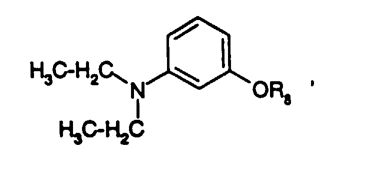 in which R₈ is hydrogen, methyl or ethyl, in a medium comprising DMF, hydrobromic acid and water,
b) without isolating the resultant nitroso compound compound, condensing it with a compound of the formula 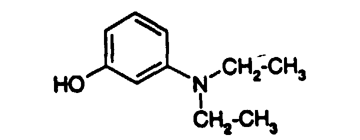 in a solvent mixture comprising DMF, hydrobromic acid and water, and
c) concentrating the reaction solution by distillation so that the dye salt is precipitated.

3. The use of the compound of formula (1b) according to claim 1 for dyeing or printing tanned cellulose fibres, silk, leather or wholly synthetic fibres.

4. The use according to claim 3 for dyeing or printing fibres comprising polyacrylonitrile or polyvinylidene cyanide.

5. The use of the compound of the formula (1b) according to claim 1 for preparing a stable liquid formulation of the compound of the formula

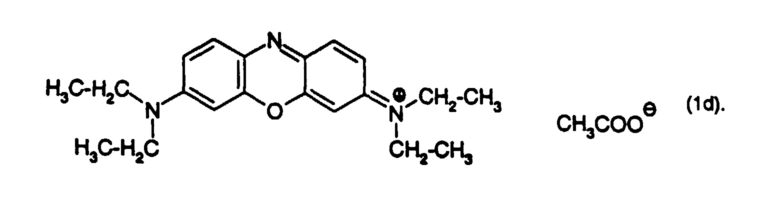

6. A process for preparing a stable liquid formulation of the compound of the formula 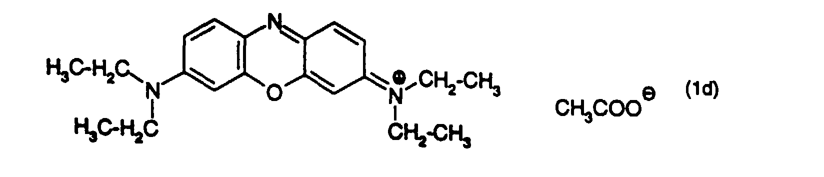 according to claim 5, which comprises transferring the moist product filter-cake obtained in the synthesis of the compound of the formula 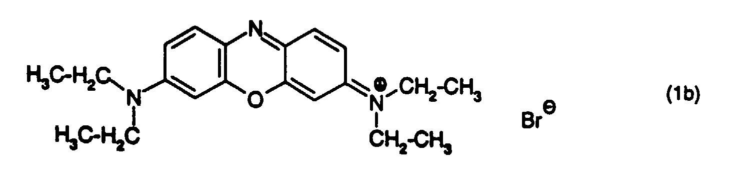 directly into a medium comprising acetic acid, sodium acetate or potassium acetate, and acetic anhydride and treating it at elevated temperature, then filtering off the precipitated alkali metal bromide and removing any excess acetic acid, and/or adjusting the colour strength of the solution by adding water.

## Revendications

1. Composé de formule

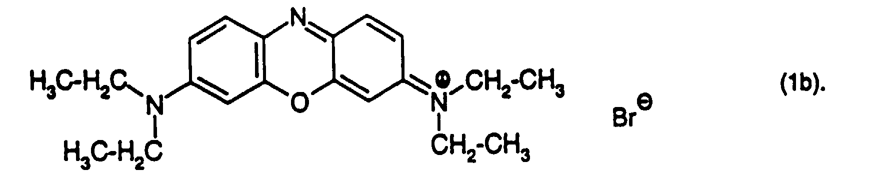

2. Procédé pour la préparation du composé de formule (1b) selon la revendication 1, caractérisé en ce qu'on réalise
a) la nitrosation d'un composé de formule 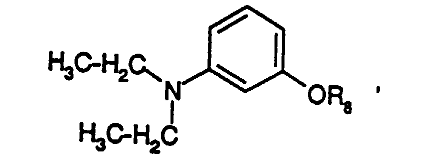 dans laquelle
R₈ représente un atome d'hydrogène, un groupe méthyle ou éthyle, dans un milieu contenant du DMF, de l'acide bromhydrique et de l'eau,
b) la condensation du composé nitroso obtenu sans séparation intermédiaire avec un composé de formule 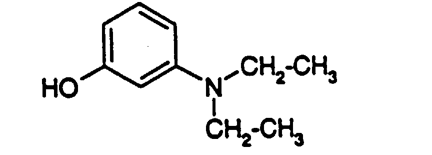 dans un mélange de solvants contenant du DMF, de l'acide bromhydrique et de l'eau, et
c) la concentration de la solution de réaction par distillation de manière à ce que le sel du colorant précipite.

3. Utilisation de composés de formule (1b) selon la revendication 1 pour la coloration ou l'impression de fibres de cellulose tannées, de soie, de cuir ou de fibres entièrement synthétiques.

4. Utilisation selon la revendication 3 pour la coloration ou l'impression de fibres contenant du polyacrylonitrile ou du polycyanure de vinylidène.

5. Utilisation de composés de formule (1b) selon la revendication 1 pour la préparation d'une formulation liquide stable du composé de formule

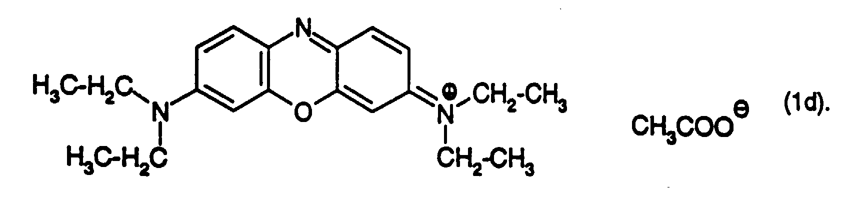

6. Procédé pour la préparation d'une formulation liquide du composé de formule 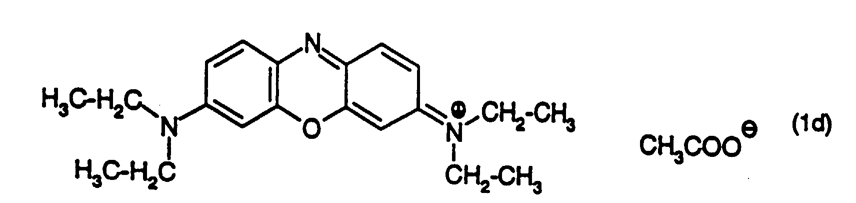 selon la revendication 5, caractérisé en ce qu'on transfère au cours de la synthèse du composé de formule 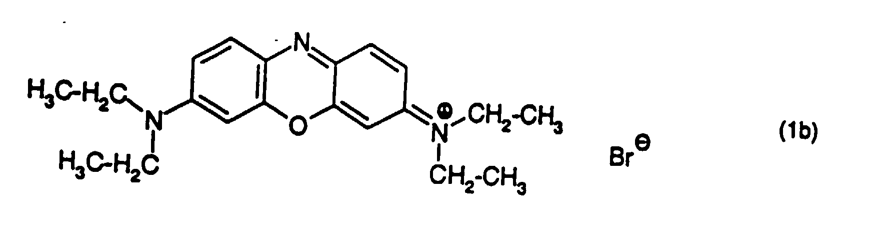 le gâteau de filtre presse humide obtenu du produit, directement dans un milieu contenant de l'acide acétique, de l'acétate de sodium ou de potassium et de l'acétanhydride et en ce qu'on le traite à température élevée, on filtre ensuite le bromure alcalin précipité et on élimine éventuellement l'acide acétique en excès et/ou on ajuste l'intensité de coloration de la solution par addition d'eau.
